# EUROPÄISCHE PATENTANMELDUNG

(11) **EP 4 273 880 A1**
(43) Veröffentlichungstag der Anmeldung: **08.11.2023**
(21) Anmeldenummer: 23171895.8
(22) Anmeldetag: 05.05.2023
(51) Int. Cl.: G16H 50/00, A61B 34/10

(54) **GENERIERUNG VON VIRTUELLEN OPTOELEKTRONISCHEN DATEN**

(30) Priorität: 06.05.2022 DE 102022111283
(71) Anmelder: Aesculap AG, 78532 Tuttlingen (DE)
(72) Erfinder: UTZ, Michael, 78532 Tuttlingen (DE); MAAS, Allan, 78467 Konstanz (DE); DUPRAZ, Ingrid, 78532 Tuttlingen (DE); VAZQUEZ URENA, Daniel Alberto, 79224 Umkirch (DE); ORTIGAS VASQUEZ, Ariana, 78467 Konstanz (DE)
(74) Vertreter: Winter, Brandl - Partnerschaft mbB

(57) **Zusammenfassung**

Die vorliegende Offenbarung betriff eine Elektronische Vorrichtung (1) zum Simulieren eines Gelenks, insbesondere eines Kniegelenks, und zum Bereitstellen kinematischer Datensätze, mit einer Simulationseinheit (7), welche dazu vorgesehen und ausgebildet ist, um ein Gelenk-Modell (2) mit zumindest einem ersten Gelenksknochen (3) und einem zweiten Gelenksknochen (4) zu erzeugen, wobei die Simulationseinheit (7) dazu vorgesehen und ausgebildet ist, um zumindest eine erste und eine zweite virtuelle Messeinheit (5) in das Gelenk-Modell (2) zu integrieren, wobei die zumindest erste virtuelle Messeinheit (5) an zumindest dem ersten Gelenksknochen (3) und die zumindest zweite virtuelle Messeinheit (5) an dem zweiten Gelenksknochen (4) angeordnet ist, und einer Recheneinheit (8), die dazu vorgesehen und ausgebildet ist, um Daten der zumindest ersten und zweiten virtuellen Messeinheiten (5) zu verarbeiten.

## Beschreibung

### Technisches Gebiet

Die vorliegende Offenbarung betrifft eine Vorrichtung und ein Computer-implementiertes Verfahren zum Simulieren eines Gelenks bzw. zum Simulieren eines Bewegungsablaufs eines Gelenks, um kinematische Datensätze zu generieren und/oder eine KI/ML-Anwendung zu trainieren.

### Hintergrund der Offenbarung

Um KI (Künstliche Intelligenz) für klinische Anwendungen zu trainieren, sind viele Patientendaten notwendig. Diese zu erheben bzw. zu bekommen ist aus unterschiedlichen Gründen schwierig, insbesondere aufgrund der Aspekte:
Ethik, Aufwand, Datenschutz, Einverständnis der Patienten, usw.
Stand heute werden Trainingsdaten für klinische ML-Anwendungen (Machine-Learning) mit großem Aufwand in Kliniken und mit Patienten gesammelt und aufgezeichnet.

Im Automotive Bereich werden heute bereits Simulationen von Verkehrssituationen verwendet, um Neuronale Netze im Bereich des autonomen Fahrens zu trainieren. D.h. das Training erfolgt hier nicht anhand realer Verkehrssituationen, sondern auf Basis von deren Simulation.

### Kurzbeschreibung der Erfindung

Es ist die objektive technische Aufgabe der vorliegenden Offenbarung, eine Vorrichtung und/oder ein Verfahren bereitzustellen, mit deren/dessen Hilfe möglichst viele Varianten kinematischer Datensätze verschiedener Gelenke auf einfache und unkomplizierte Weise generierbar sind.

Die vorstehende Aufgabe wird durch den Offenbarungsgegenstand des Anspruchs 1 gelöst.

Im Konkreten wird die vorstehende Aufgabe gelöst durch eine Elektronische Vorrichtung zum Simulieren eines Gelenks, insbesondere eines Kniegelenks, und zum Bereitstellen kinematischer Datensätze, mit einer Simulationseinheit, welche dazu vorgesehen und ausgebildet ist, um ein Gelenk-Modell mit zumindest einem ersten Gelenksknochen und einem zweiten Gelenksknochen zu erzeugen, wobei die Simulationseinheit dazu vorgesehen und ausgebildet ist, um zumindest eine erste virtuelle, vorzugsweise inertiale, und eine zweite virtuelle, vorzugsweise inertiale, Messeinheit in das Gelenk-Modell zu integrieren, wobei die zumindest erste virtuelle Messeinheit an zumindest dem ersten Gelenksknochen und die zumindest zweite virtuelle Messeinheit an dem zweiten Gelenksknochen angeordnet ist, und einer Recheneinheit, die dazu vorgesehen und ausgebildet ist, um Daten der zumindest ersten und zweiten virtuellen Messeinheiten zu verarbeiten. Unter einer Simulation ist die Nachbildung von Dingen bzw. Gegenständen oder Prozessen zu verstehen. Somit ist die Simulation eines Gelenks die Darstellung des Gelenks als solches sowie die Bewegung, zu welcher das Gelenk fähig ist.

In nochmals anderen Worten wird mit der Verwendung von ergänzenden/ zusätzlichen virtuellen Messeinheiten an den beiden das Gelenk bildenden Gelenksknochen eine Lösung dafür bereitgestellt, um in Abwesenheit von Patienten kinematische Datensätze zu generieren, mit welchen Kls und/oder ML-Anwendungen trainiert werden können. Dies hat den technischen Effekt, dass mit relativ geringem Zeitaufwand in kurzer Zeit viele Datensätze mit hoher Varianz generierbar sind. Die offenbarungsgemäßen virtuellen Messeinheiten sind vorzugsweise als virtuelle inertiale Messeinheiten ausgebildet, beispielsweise in der Form von IMUs (Inertial Measuring Units). Alternativ oder zusätzlich, sind die Messeinheiten bspw. als reflektierende Marker/ Markierungen/ Kennzeichnungen ausgebildet, welche auf die Haut geklebt bzw. über Hülsen angebracht werden. Auf diese Weise sind virtuelle optoelektronische Daten oder Informationen über Position(en) starrer Körper generierbar. Dadurch ist die Weichteilsituation zusätzlich zur Knochenbewegung simulierbar. Eine weitere Alternative oder zusätzliche Möglichkeit ist die Erzeugung/ Generierung von Daten aus Magnetometer(n) und Beschleunigungssensor(en) (durch die Messung von Beschleunigung, Winkelgeschwindigkeit und/oder des Magnetfelds).

Somit bietet die vorliegende Offenbarung einen Weg, durch die Nutzung von validierten Simulationstools/ einer validierten Simulationsvorrichtung solche Daten in der notwendigen Anzahl und mit der gewünschten Varianz zu bekommen/zu generieren. Daher wird beispielsweise ein kinematisches Kniemodell dazu verwendet, um theoretische Daten der eingesetzten virtuellen (inertialen) Messeinheiten zu generieren.

Es ist von Vorteil, wenn die Vorrichtung dazu vorgesehen und ausgebildet ist, um damit Bewegungsabläufe, Relativposition(en) und eine Orientierung des zumindest ersten Gelenksknochens und des zweiten Gelenksknochens verschiedener Patienten zu simulieren.

Es ist bevorzugt, wenn die Vorrichtung dazu vorgesehen und ausgebildet ist, um anhand von Daten der zumindest zwei virtuellen Messeinheiten auf eine relative Position von dem ersten Gelenksknochen zu dem zweiten Gelenksknochen rückzuschließen.

Es ist von Vorteil, wenn eine Vielzahl von Parametern sowie deren Kombinationen variierbar und simulierbar sind. Dies bietet die Möglichkeit Gelenke verschiedener Patienten mit unterschiedlichen Problematiken, unterschiedlichen körperlichen Begebenheiten zu simulieren und Datensätze zu erhalten.

Es ist vorteilhaft, wenn folgende Parameter wählbar bzw. miteinander beliebig kombinierbar sind:
- unterschiedliche Lastfälle, insbesondere Extension und/oder Flexion mit und ohne Belastung, vorzugsweise in unterschiedlichen Winkeln des Gelenks
- Anatomische Varianten des Modells, insbesondere Varus oder Valgus oder Slope oder Phänotyp
- Patienten- und entsprechende Knochengröße
- Patientendaten, insbesondere Gewicht und/oder BMI und/oder Weichteildeckung/-situation
- Position der virtuellen Messeinheiten an dem Gelenk
- Orientierung der virtuellen Messeinheiten

Unter unterschiedlichen Lastfällen sind beispielsweise ein ebenes Gehen, ein Treppensteigen und/oder beispielsweise Physiotherapieübungen zu verstehen.

In anderen Worten sind die vorstehend genannten Parameter einstellbar und beliebig miteinander kombinierbar, wodurch eine hohe Anzahl an Datensätzen basierend auf unterschiedlichen Ausgangssituationen erreichbar/generierbar ist. Das heißt eine Vielzahl der Parameter sowie deren zahlreiche Kombinationen, variiert und simuliert werden.

Es ist bevorzugt, wenn bei jedem Simulationszyklus Ausgangsdaten der virtuellen Messeinheiten berechenbar und die Vorrichtung dazu vorgesehen ist, aus den Ausgangsdaten resultierende Datensätze auszugeben.

In anderen Worten werden bei jedem Simulationszyklus Ausgangsdaten der virtuellen (inertialen) Messeinheiten berechnet. Durch die Varianz der vorstehend genannten Parameter können Ausgangsdaten der virtuellen (inertialen) Messeinheiten für eine Vielzahl von virtuellen Patienten und Installationen von virtuellen Messeinheiten/Messeinheit-Setups generiert werden.

Hierbei ist es weiter von Vorteil, wenn Ausgangsdaten zu jeder virtuellen, vorzugsweise inertialen, Messeinheit über die Zeit und über Flexion und/oder Extension des Gelenks ausgebbar sind. In anderen Worten können die Ausgangsdaten zu jeder virtuellen, vorzugsweise inertialen, Messeinheit nicht nur über die Zeit, wie bei echten inertialen Messeinheiten, ausgegeben werden, sondern der Datensatz kann auch über die Flexion und/oder Extension ausgegeben werden.

Es ist vorteilhaft, wenn die aus den Ausgangsdaten resultierenden Datensätze zum Trainieren von Neuronalen Netzen, insbesondere KI und/oder ML-Anwendungen, vorgesehen sind. In anderen Worten bedeutet das, dass diese virtuellen Messeinheiten-Ausgangsdatensätze anschließend für das Training von Neuronalen Netzen genutzt werden, mit dem Effekt/Ziel, anhand der virtuellen Messeinheiten-Daten, rückwärts, auf den Werteverlauf der Eingangsparameter schließen zu können.

Es ist bevorzugt, wenn die Vorrichtung dazu vorgesehen und ausgebildet ist, um durch weitere knöcherne Strukturen, vorzugsweise das nächstgelegene Gelenk, erweiterbar zu sein. In anderen Worten kann die Simulationsvorrichtung/ das Simulationsmodell um weitere knöcherne Strukturen erweitert werden. Zum Beispiel kann zu Femur und Tibia auch das Becken hinzugenommen werden. Diese Methode lässt sich auch auf andere Gelenke, wie später beschrieben, übertragen.

Zusammengefasst lässt sich mit Hilfe der vorstehend beschriebenen Simulationsvorrichtung mit relativ geringem Aufwand in kurzer Zeit viele Datensätze mit hoher Varianz generieren. Hierbei ist es ein wesentliches Ziel, auf die relative Position von dem zumindest einen ersten Gelenksknochen, vorzugsweise von Tibia, zu dem zweiten Gelenksknochen, vorzugsweise zu Femur, anhand der erhaltenen Daten durch die virtuellen (inertialen) Messeinheiten schließen zu können.

Ferner betrifft die vorliegende Offenbarung die Verwendung der Vorrichtung gemäß den vorstehenden Aspekten bei Hüftgelenk, Schultergelenk, Ellenbogengelenk, Kniegelenk, Fußgelenk, Handgelenk und/ oder Wirbelsäule. In anderen Worten kann dieses Vorgehen grundsätzlich auf andere, simulierbare Vorgänge im menschlichen Körper übertragen werden, bei denen über Sensordaten auf physiologische Vorgänge geschlossen werden soll.

Ferner betrifft die vorliegende Offenbarung ein Computer-implementiertes Verfahren zum Simulieren, Trainieren und Validieren eines Gelenk-Modells, insbesondere eines Kniegelenk-Modells, mit folgenden Schritten:
- Bereitstellen eines validierten, virtuellen Gelenkmodells;
- Integration von virtuellen (inertialen) Messeinheiten;
- Ausgabe und Verarbeitung von Datensätzen basierend auf den Daten der virtuellen Messeinheiten;
- Trainieren einer ML-Anwendung basierend auf den ausgegebenen Datensätzen aus dem vorhergehenden Schritt; und
- Validieren der ML-Anwendung.

In anderen Worten werden in einem ersten Schritt bei der Bereitstellung eines validierten, virtuellen Gelenkmodells mögliche Variantensimulationen eingespielt. Dies betrifft die Einstellung der Parameter, beispielsweise die Anatomie, den Lastfall, den BMI und/oder die Weichteilsituation.

In dem darauffolgenden Schritt werden die virtuellen (inertialen) Messeinheiten an dem ersten Gelenksknochen und dem zweiten Gelenksknochen integriert. Durch die Integration der virtuellen Messeinheiten werden Position, Orientierung und Typ berücksichtigt.

In einem nächsten Schritt erfolgt die Erfassung und Ausgabe der Daten, welche aus den virtuellen (inertialen) Messeinheiten erhalten/generiert werden. Dabei handelt es sich um Messeinheiten-Daten und/oder kinematische Daten und/oder einem aus den erhaltenen Daten abgeleiteten Phänotyp.

Darauf folgt ein Schritt, in welchem KI/ ML-Anwendungen trainiert werden. Das Training bezieht sich auf die Kinematik, den Phänotyp und die Relativposition sowie Orientierung von dem ersten Gelenksknochen zu dem zweiten Gelenksknochen, bspw. Femur zu Tibia.

In einem letzten Schritt gilt es die KI/ ML-Anwendung gegenüber der Simulation und gegenüber zumindest einem Patienten zu validieren.

Ferner betrifft die vorliegende Offenbarung ein computerlesbares Speichermedium, welches die Befehle umfasst, die bei einer Ausführung durch die vorstehende Vorrichtung diese veranlassen, das Verfahren gemäß dem vorstehenden Aspekt auszuführen.

Kurzbeschreibung der Figuren
Fig. 1 ist eine Darstellung zur Veranschaulichung einer Vorrichtung gemäß einer Ausführung der vorliegenden Offenbarung; und
Fig. 2 ist eine Darstellung zur Veranschaulichung eines Ablaufs gemäß einer Ausführungsform der vorliegenden Offenbarung.

### Beschreibung der Ausführungsform

Nachstehend werden Ausführungsbeispiele der vorliegenden Offenbarung auf der Basis der zugehörigen Figuren beschrieben.

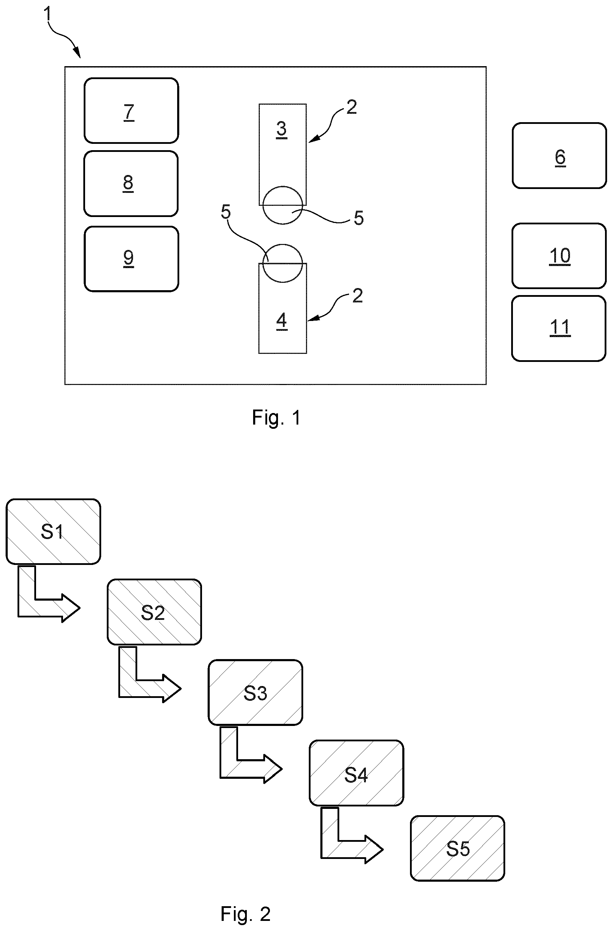

Fig. 1 ist eine Darstellung zur Veranschaulichung einer Vorrichtung 1 gemäß einer Ausführung der vorliegenden Offenbarung. Fig. 1 zeigt die elektronische Vorrichtung 1 zum Simulieren eines Gelenk, insbesondere eines Kniegelenks, und zum Bereitstellen kinematischer Datensätze.

Die Vorrichtung 1 hat eine Simulationseinheit 7, welche dazu vorgesehen und ausgebildet ist, um ein Gelenk-Modell 2 mit zumindest einem ersten Gelenksknochen 3 und einem zweiten Gelenksknochen 4 zu erzeugen. Die Simulationseinheit 7 ist dazu vorgesehen und ausgebildet, um zumindest eine erste und eine zweite virtuelle, vorzugsweise inertiale, Messeinheit 5 in das Gelenk-Modell 2 zu integrieren. Die zumindest erste virtuelle, vorzugsweise inertiale, Messeinheit 5 ist an zumindest dem ersten Gelenksknochen 3 und die zumindest zweite virtuelle, vorzugsweise inertiale, Messeinheit 5 ist an dem zweiten Gelenksknochen 4 angeordnet ist.

Fig. 1 zeigt eine Recheneinheit 8 sowie eine Messvorrichtung 9. Die Messvorrichtung 9 ist dazu vorgesehen und ausgebildet, um die zumindest eine erste und zweite Messeinheit 5 zu integrieren und zu verwenden. Die Recheneinheit 8 ist vorgesehen und ausgebildet, um Daten der zumindest ersten und zweiten virtuellen, vorzugsweise inertialen, Messeinheiten 5 zu verarbeiten. Die Anordnung der virtuellen Messeinheiten 5 an dem ersten Gelenksknochen 3 und dem zweiten Gelenksknochen 4 ist lediglich beispielhaft. Fig. 1 zeigt auch eine Trainingseinheit 10 und eine Validierungseinheit 11, die zum Trainieren und Validieren neuronaler Netze auf der Grundlage der von der Vorrichtung 1 ausgegebenen Daten vorgesehen und konfiguriert sind.

Fig. 2 ist eine Darstellung zur Veranschaulichung eines Ablaufs gemäß einer Ausführungsform der vorliegenden Offenbarung. In einem ersten Schritt S1 werden bei der Bereitstellung eines validierten, virtuellen Gelenkmodells 2 mögliche Variantensimulationen eingespielt. Dies betrifft die Einstellung der Parameter, beispielsweise die Anatomie, den Lastfall, den BMI und/oder die Weichteilsituation.

In einem Schritt S2 werden die virtuellen, vorzugsweise inertialen, Messeinheiten 5 an dem ersten Gelenksknochen 3 und dem zweiten Gelenksknochen 4 integriert. Durch die Integration der virtuellen Messeinheiten 5 werden Position, Orientierung und Typ berücksichtigt.

In einem nächsten Schritt S3 erfolgt die Erfassung und Ausgabe der Daten, welche aus den virtuellen, vorzugsweise inertialen Messeinheiten 5 erhalten/generiert werden. Dabei handelt es sich um inertiale Messeinheiten-Daten und/oder kinematische Daten und/oder einem aus den erhaltenen Daten abgeleiteten Phänotyp.

Darauf folgt ein Schritt S4, in welchem das neuronale Netz, insbesondere KI/ ML-Anwendungen 6 mittels einer Trainingseinheit 10 trainiert wird/ werden. Das Training bezieht sich auf die Kinematik, den Phänotyp und die Relativposition sowie Orientierung von dem ersten Gelenksknochen 3 zu dem zweiten Gelenksknochen 4, bspw. Femur zu Tibia.

In einem letzten Schritt S5 gilt es das neuronale Netz/ die KI/ ML-Anwendung 6 gegenüber der Simulation und gegenüber zumindest einem Patienten mittels einer Validierungseinheit 11 zu validieren.

### Bezugszeichenliste

- 1: Vorrichtung
- 2: Gelenk-Modell
- 3: Erster Gelenksknochen
- 4: Zweiter Gelenksknochen
- 5: Messeinheiten
- 6: KI/ML-Anwendung
- 7: Simulationseinheit
- 8: Recheneinheit
- 9: Messvorrichtung
- 10: Trainingseinheit
- 11: Validierungseinheit

## Patentansprüche

1. Elektronische Vorrichtung (1) zum Simulieren eines Gelenks, insbesondere eines Kniegelenks, und zum Bereitstellen kinematischer Datensätze, mit:
einer Simulationseinheit (7), welche dazu vorgesehen und ausgebildet ist, um ein Gelenk-Modell (2) mit zumindest einem ersten Gelenksknochen (3) und einem zweiten Gelenksknochen (4) zu erzeugen, wobei die Simulationseinheit (7) dazu vorgesehen und ausgebildet ist, um zumindest eine erste und eine zweite virtuelle Messeinheit (5), vorzugsweise eine erste und eine zweite virtuelle, inertiale Messeinheit (5), in das Gelenk-Modell (2) zu integrieren, wobei die zumindest erste virtuelle Messeinheit (5) an zumindest dem ersten Gelenksknochen (3) und die zumindest zweite virtuelle Messeinheit (5) an dem zweiten Gelenksknochen (4) angeordnet ist, und
einer Recheneinheit (8), die dazu vorgesehen und ausgebildet ist, um Daten der zumindest ersten und zweiten virtuellen Messeinheiten (5) zu verarbeiten.

2. Elektronische Vorrichtung (1) gemäß Anspruch 1, **dadurch gekennzeichnet, dass** die Vorrichtung (1) dazu vorgesehen und ausgebildet ist, um anhand von Daten der zumindest zwei virtuellen Messeinheiten (5) auf eine relative Position von dem ersten Gelenksknochen (3) zu dem zweiten Gelenksknochen (4) rückzuschließen.

3. Elektronische Vorrichtung (1) gemäß Anspruch 1 oder 2, **dadurch gekennzeichnet, dass** eine Vielzahl von Parametern sowie deren Kombinationen variierbar und simulierbar sind.

4. Elektronische Vorrichtung (1) gemäß Anspruch 3, **dadurch gekennzeichnet, dass** folgende Parameter wählbar bzw. miteinander beliebig kombinierbar sind:
- unterschiedliche Lastfälle, insbesondere Extension und/oder Flexion mit und ohne Belastung, vorzugsweise in unterschiedlichen Winkeln des Gelenks
- Anatomische Varianten des Modells, insbesondere Varus oder Valgus oder Slope oder Phänotyp
- Patienten- und entsprechende Knochengröße
- Patientendaten, insbesondere Gewicht und/oder BMI und/oder Weichteildeckung/-situation
- Position der virtuellen Messeinheiten (5) an dem Gelenk
- Orientierung der virtuellen Messeinheiten (5)

5. Elektronische Vorrichtung (1) gemäß einem vorhergehenden Anspruch,
**dadurch gekennzeichnet, dass** bei einem Simulationszyklus Ausgangsdaten der virtuellen Messeinheiten (5) berechenbar und die Vorrichtung (1) dazu vorgesehen ist, aus den Ausgangsdaten resultierende Datensätze auszugeben.

6. Elektronische Vorrichtung (1) gemäß Anspruch 5, **dadurch gekennzeichnet, dass** Ausgangsdaten zu jeder virtuellen Messeinheit (5) über die Zeit und über Flexion und/oder Extension des Gelenks (2) ausgebbar sind.

7. Elektronische Vorrichtung (1) gemäß Anspruch 5 oder 6, **dadurch gekennzeichnet, dass** die aus den Ausgangsdaten resultierenden Datensätze zum Trainieren von Neuronalen Netzen, insbesondere KI und/oder ML-Anwendungen (6), vorgesehen sind.

8. Elektronische Vorrichtung (1) gemäß einem der vorhergehenden Ansprüche, **dadurch gekennzeichnet, dass** die Vorrichtung (1) dazu vorgesehen und ausgebildet ist, um die Simulation des Gelenks (3) durch weitere knöcherne Strukturen, vorzugsweise das dem Gelenk (3) nächstgelegene Gelenk, erweiterbar zu sein.

9. Verwendung der Vorrichtung (1) gemäß Ansprüche 1 bis 8 zum Simulieren eines Hüftgelenks, Schultergelenks, Ellenbogengelenks, Kniegelenks, Fußgelenks, Handgelenks und/ oder einer Wirbelsäule.

10. Computer-implementiertes Verfahren zum Simulieren eines Gelenk-Modells, insbesondere eines Kniegelenk-Modells, sowie zum , Trainieren und Validieren eines neuronalen Netzes, mit folgenden Schritten, welche mittels der Vorrichtung (1) gemäß den Ansprüche 1 bis 8 durchgeführt werden:
- Bereitstellen eines validierten, virtuellen Gelenkmodells (S1) mittels der Vorrichtung (1);
- Integration von virtuellen Messeinheiten (S2) in das virtuelle Gelenkmodell mittels einer Messvorrichtung (9);
- Ausgabe und Verarbeitung von Datensätzen basierend auf den von den virtuellen Messeinheiten (S3) ausgegebenen Daten mittels einer Recheneinheit (8) der Vorrichtung (1);
- Trainieren von neuronalen Netzen, insbesondere einer ML-Anwendung (S4) basierend auf den ausgegebenen Datensätzen aus dem vorhergehenden Schritt mittels einer Trainingseinheit (10); und
- Validieren der ML-Anwendung (S5) mittels einer Validierungseinheit (11).

11. Computerlesbares Speichermedium, das Befehle umfasst, die bei einer Ausführung durch eine Vorrichtung (1) gemäß den Ansprüchen 1 bis 8 diese veranlassen, das Verfahren nach Anspruch 10 auszuführen.
